# EUROPÄISCHE PATENTSCHRIFT

(11) **EP 1 393 759 B1**
(45) Veröffentlichungstag und Bekanntmachung des Hinweises auf die Patenterteilung: **26.10.2005**
(21) Anmeldenummer: 03017808.1
(22) Anmeldetag: 05.08.2003
(51) Int. Cl.: A61L 33/06, A61L 29/04

(54) **Medizinisches Arbeitsmittel auf Basis von Weich-PVC**
Medical article on the basis of plasticized PVC
Article médical en PVC mou

(30) Priorität: 29.08.2002 DE 10239737
(43) Veröffentlichungstag der Anmeldung: 03.03.2004
(73) Patentinhaber: RAUMEDIC AG, 95233 Helmbrechts (DE)
(72) Erfinder: Heinlein, Martin, 95111 Rehau (DE); Kühlein, Georg, 95111 Rehau (DE)
(74) Vertreter: Hofmann, Matthias

(56) Entgegenhaltungen:
- EP-A- 0 141 471
- EP-A- 0 397 326
- WO-A-03/029339
- DE-A- 3 444 155
- DE-A- 19 927 977
- DE-C- 10 129 129
- DE-U- 20 021 356
- US-A- 3 218 289
- US-A- 6 060 138
- F.A.Z.-INSTITUT FÜR MANAGEMENT-, MARKT- UND MEDIENINFORMATIONEN GMBH: "BASF entwickelt Weichmacher für sensible Anwendungen" FAZ-INSTITUT, CHEMICAL NEWSFLASH, NEWS VOM 2.JULI BIS ZUM 8.JULI 2002, [Online] XP002263914 Gefunden im Internet: <URL:http://www.chemicalnewsflash.de/de/ne ws/090702/news8.htm> [gefunden am 2003-12-03]

## Beschreibung

Die Erfindung betrifft ein Verfahren zur Herstellung von medizinischen Schläuchen, wobei eine Alternative zu Di(2-ethylhexyl)phtalat (DEHP) als Weichmacher verwendet wird. Ferner betrifft die Erfindung Verwendungen der so hergestellten medizinischen Schläuche.

Weich-PVC - und damit auch DEHP - wird außerordentlich vielseitig eingesetzt, vom Bereich Automobilindustrie über Anwendungen im Haushalt bis hin zur Medizin.
Allerdings stehen die Medizinanwendungen von Weich-PVC seit vielen Jahren in der Kritik, vor allem, da die DEHP grundsätzlich - besonders aber in Kontakt mit Blut - ausgewaschen wird und damit eine schädliche Auswirkung auf die Patientengesundheit befürchtet wird.

Zur Lösung dieses Problems ging die Forschung in den vergangenen 30 Jahren folgende zwei Wege:
a) Entwicklung nicht extraktionsfähiger Weichmacher
b) Untersuchungen zur akuten und chronischen Toxizität von DEHP

Im ersteren Fall gelang es beispielsweise, die Weichmachermigration drastisch, in etwa um den Faktor 100, durch die Entwicklung von Polymerweichmachern zu reduzieren.
In der Praxis waren die Polymerweichmacher jedoch zu teuer und zeigten zusätzlich den Nachteil, dass nur Shore-Härten ab A 70 und härter einstellbar sind.

Auch Monomerweichmacher mit einem hohen Molekulargewicht, wie das Tri(2-ethylhexyl)trimellitat zeigten vielversprechende Ansätze, allerdings setzte sich auch diese Substanz trotz einer ebenfalls um dem Faktor 100 reduzierten Weichmachermigration aus Kostengründen in der Praxis nicht durch.

Die Untersuchungsergebnisse zur Toxizität von DEHP waren trotz eines außerordentlich hohen Aufwandes nicht eindeutig. Im letzten Jahr verstärkten sich allerdings die Hinweise, dass DEHP sehr wahrscheinlich doch reproduktionstoxisch wirkt.

Nach wie vor war also das Problem ungelöst, einen Weichmacher für PVC im Medizinbereich bereitzustellen, der nicht nur nicht toxisch, sondern auch kostengünstig sein sollte, der auf den üblichen PVC-Aufbereitungs- bzw. -Verarbeitungsmaschinen verarbeitbar ist und der keine oder nur geringfügige Rezepturmodifikationen nötig macht, um die bekannten Anwendungen in der notwendigen Qualität zu realisieren.

In der Internet-Veröffentlichung "BASF entwickelt Weichmacher für sensible Anwendungen", Chemical News Flash, News vom 2. Juli bis 8. Juli 2002, abrufbar am 3.12.2002, ist ein PVC-Weichmacher mit der Produktbezeichnung Hexamoll DINCH bekannt. Als Anwendungen für diesen Weichmacher sind Lebensmittelfolien, medizinische Artikel, wie beispielsweise Infusionsschläuche, und Spielzeug aus PVC genannt.

Die DE 34 44 155 A1 nennt eine Zusammensetzung auf dem Gebiet der Weich-PVC-Verarbeitung mit 60 bis 70 Gew.-% PVC, 30 bis 40 Ges.-% Weichmacher, 2 bis 10 % eines epoxidierten Weichmacheröls, wie epoxidiertes Sojabohnenöl, bis zu 2 % eines Gleitmittels und bis zu 1 % eines Stabilisators, wie Zink- und/oder Kalziumstearat.

Die DE 200 21 356 U1 offenbart PVC mit einem Cyclohexan-1,2-Dicarbonsäure-diisononylester als Weichmacher für die Anwendung in medizinischen Artikeln.

Mit der Synthese von Di(isononyl)-Cyclohexan-1,2-Dicarboxylat (Produktbezeichnung Hexamoll DINCH der BASF AG, Ludwigshafen, technische Unterlage vom 5.3.2002) ist nun seit kurzem eine Substanz verfügbar, die in den bisherigen Tests weder akute noch chronische Toxizität noch Gentoxizität zeigt, die biologisch abbaubar ist und keine Hautreizungen hervorruft.

Hexamoll DINCH (nachfolgend als DINCH bezeichnet) wurde insbesondere für Anwendungen mit menschlichem Kontakt entwickelt, also für Kinderspielzeug, Medizinalprodukte und Lebensmittelverpackungen.

Die wichtigsten Anwendungen von PVC mit DINCH als Weichmacher im Medizinbereich sind grundsätzlich Schläuche für die Dialyse, Drainage und Infusion. Gerade bei der Herstellung dieser Artikel zeigte sich jedoch, dass - entgegen den Angaben in der oben zitierten BASF-Broschüre - geringfügige Anpassungen von Rezepturen und Verarbeitungsverfahren keinesfalls genügen, um das Produkt problemlos und in der gewünschten Qualität auf den vorhandenen Verarbeitungsmaschinen verarbeiten zu können.

Es ergab sich daher die Aufgabenstellung, besonders für transparente Schläuche, deren Shore-Härten im Bereich von A 75 bis A 87 liegen und die über eine spezielle Oberflächentextur verfügen müssen, und zwar
- glatt/hochglänzend (für blutführende Systeme)
- matt und gleitfähig (für Pumpsegmente)
- mikrorauh (für Schläuche, die mit Konfektionsautomaten verarbeitet werden)
geeignete rezepturseitige und verfahrenstechnische Bedingungen zu finden, mit denen die bekannten Anforderungen an Schläuche für die Dialyse, Infusion und Drainage erfüllt werden können.

Die Lösung der Aufgabe gelang durch eine Kombination geeigneter Aufbereitungsbedingungen in Verbindung mit verfahrenstechnischen Parametern und rezepturseitigen Maßnahmen, die in dieser Zusammensetzung nicht aus dem BASF-Merkblatt und dem Wissen, das sich aus dem Stand der Technik ergibt, herleitbar sind.
Dies soll nachfolgend - bei der Beschreibung der Erfindung - näher erläutert werden.

Die Lösetemperatur für PVC beträgt im Falle von DINCH 151°C, im Falle von DEHP 124°C. Das bedeutet, dass DINCH im Schnellmischer bei ca 175C° verarbeitet werden muss. Da bei DEHP 145°C üblich sind, ist keiner der heute handelsüblichen Schnellmischer in der Lage, eine PVC-Rezeptur bei 175°C zu verarbeiten.
Im Falle von Spritzgussteilen (Beispiel Kinderspielzeug) ist dieses Temperaturdefizit von ca. 30°C nicht kritisch, da die Teile ohnehin eingefärbt werden.
Für die erfindungsgemäße Aufgabenstellung, nämlich transparente bis brillante Schläuche zu fertigten, müssen die 175°C Mischtemperatur sicher erreicht werden, um Einschlüsse und andere optische Unreinheiten zuverlässig zu vermeiden.
Das bedeutet einerseits den Umbau des üblichen PVC-Mischers in Richtung niedrige Mischleistung, jedoch höhere Mischungstemperatur, andererseits die Notwendigkeit einer wesentlichen Erhöhung der Thermostabilität der PVC-Rezeptur. Dies gilt vor allem für den Mischprozess, da hier - anders als bei der Formgebung (hier sind es 2 - 3 Minuten) - die PVC-Rezeptur über einen Zeitraum von 25 Min. thermisch belastet wird.
Eine derartige Erhöhung der Thermostabilität von PVC-Rezepturen, die für medizinische Zwecke geeignet sind, ist nicht Stand der Technik. Diese extreme Stabilisierung gelingt nur, wenn ein hoher Anteil an epoxidierten Ölen als Costabilisator mit Calcium-/Zink-Stearaten als Metallstabilisatoren in Verbindung mit Amidwachs (Bisstearylethylendiamin) synergistisch zusammenwirkt.

Nicht nur bezüglich der Aufbereitung der PVC-Rezepturen mit DINCH als Weichmacher, sondern auch bezüglich der Formgebung bei der Extrusion, genügt der Stand der Technik bzw. die Aussagen der oben zitierten BASF-Broschüre keinesfalls. Besonders wichtig für die Ausführung der gewünschten Transparenz des Schlauches bzw. Brillanz der Schlauchoberfläche sind erfindungsgemäß die Druckverhältnisse im Extrusionswerkzeug.
Diese gewünschten Eigenschaften von Schlauch bzw. Schlauchoberfläche werden bei hohen Extrusiönsdrücken, das bedeutet 150 bar und darüber, erreicht, vorzugsweise dadurch dass im Extrusionswerkzeug ein breiter Förderkanal um 90° umgelenkt und anschließend auf höchstens ein Drittel des ursprünglichen Querschnittes reduziert wird.

Nachfolgend wird die Erfindung an drei Ausführungsbeispielen im Detail erläutert:

### 1. Ausführungsbeispiel - Schläuche für Konfektionsautomaten

Die Zusammensetzung umfasst folgende Komponenten:

| | |
|---|---|
| 100 T | PVC mit K-Wert 70 |
| 47 T | Hexamoll DINCH |
| 16 T | epoxidiertes Sojaöl |
| 0,17 T | Zinkstearat |
| 0,12 T | Calciumstearat |
| 0,4 T | Bisstearylethylendiamin |

Die Mischung wird, wie oben beschrieben, im PVC-Schnellmischer bei 175°C aufbereitet, im Doppelschneckenextruder granuliert und schließlich das Granulat auf einem Einschneckenextruder, wie ebenfalls beschrieben, zum Schlauch urgeformt.

Der so erhaltene Schlauch verfügt über eine mikrorauhe Oberfläche, seine Shore-Härte beträgt ca. A 80.

### 2. Ausführungsbeispiel - Schläuche für extrakorporale, blutführende Systeme

Die Zusammensetzung umfasst folgende Komponenten:

| | |
|---|---|
| 100 T | PVC mit K-Wert 70 |
| 54 T | Hexamoll DINCH |
| 17 T | epoxidiertes Sojaöl |
| 0,20 T | Zinkstearat |
| 0,10 T | Calciumstearat |
| 0,4 T | Bisstearylethylendiamin |
| +optional | Ultramarinblaupigmente |

Aufbereitung und Urformung siehe Ausführungsbeispiel 1.
Der so erhaltene Schlauch verfügt über eine glänzende, brillante Oberfläche; seine Shorehärte beträgt ca. A 75.

### 3. Ausführungsbeispiel - Schläuche für Pumpsegmente

Die Zusammensetzung umfasst folgende Komponenten:

| | |
|---|---|
| 100 T | PVC mit K-Wert 80 |
| 63 T | Hexamoll DINCH |
| 18 T | epoxidiertes Sojaöl |
| 0,20 T | Zinkstearat |
| 0,08 T | Calciumstearat |
| 0,8 T | Bisstearylethylendiamin |

Aufbereitung und Urformung siehe Ausführungsbeispiel 1.
Der so erhaltene Schlauch verfügt über eine matte/gleitfähige Oberfläche; seine Shore-Härte beträgt A 70.

## Patentansprüche

1. Verfahren zur Herstellung von medizinischen Schläuchen, insbesondere für die Dialyse, Infusion und Drainage, auf Basis Weich-PVC mit Di(isononyl)-Cyclohexan-1,2-Dicarboxylat als Weichmacher und einem Shore-Härte-Bereich von A 65 bis A 87, wobei
die polymere Zusammensetzung umfasst
- 100 T PVC mit einem K-Wert zwischen 65 und 100
- 35 - 75 T Di(isononyl)-Cyclohexan-1,2-Dicarboxylat
- 0,05 - 0,4 T Calcium-Stearat
- 0,05 - 0,4 T Zink-Stearat
- 10 - 18 T epoxidiertes Pflanzenöl
- 0,5 - 1,5 T Amidwachs
wobei
- die Aufbereitung der Zusammensetzung bei 165 bis 180° C erfolgt,
- die Urformung der Zusammensetzung mittels eines Extrusionswerkzeugs bei einem Düsendruck von 150 bar und darüber erfolgt.

2. Verfahren nach Anspruch 1, **dadurch gekennzeichnet, dass** die Urformung der Zusammensetzung mittels eines abgewinkelten, im Durchmesser reduzierten Extrusionswerkzeuges erfolgt.

3. Verwendung von nach Anspruch 1 oder 2 hergestellten medizinischen Schläuchen, insbesondere als transparente Schläuche für blutführende Systeme, für Pumpsegmente und für die Verarbeitung auf Konfektionsautomaten.

## Claims

1. A method of manufacturing medical tubes, in particular for dialysis, infusion and drainage, on the basis of plasticized PVC with di(isononyl)-cyclohexane-1,2-dicarboxylate as plasticizer and a Shore hardness range of A 65 to A 87,
the polymeric composition comprising
- 100 T PVC of a K value between 65 and 100
- 35 - 75 T di(isononyl)-cyclohexane-1,2-dicarboxylate
- 0.05 - 0.4 T calcium stearate
- 0.05 - 0.4 T zinc stearate
- 10 - 18 T epoxidized vegetable oil
- 0.5 - 1.5 T amide wax
wherein
- preparation of the composition takes place at 165 to 180°C
- primary forming of the composition is effected by means of an extrusion die at, and above, a die pressure of 150 bar.

2. A method according to claim 1, **characterized in that** the primary forming of the composition takes place by means of an elbowed extrusion die of reduced diameter.

3. Use of the medical tubes manufactured according to claim 1 or 2, in particular as transparent tubes for blood-conveying systems, for pump segments and for processing on automatic assembly machines.

## Revendications

1. Procédé de fabrication de tuyaux médicaux, en particulier pour la dialyse, l'infusion et le drainage, à base de PVC mou avec du 1,2-dicarboxylate de di(isononyl)-cyclohexane en tant que plastifiant et un domaine de dureté Shore de A 65 à A 87, sachant que la composition polymère comprend
- 100 parties de PVC ayant une valeur K entre 65 et 100
- 35-75 parties de 1,2-dicarboxylate de di(isononyl)-cyclohexane
- 0,05-0,4 parties de stéarate de calcium
- 0,05-0,4 parties de stéarate de zinc
- 10-18 parties d'huile végétale époxydée
- 0,5-1,5 parties de cire d'amide
sachant que
- la préparation de la composition s'effectue entre 165 et 180°C,
- la mise en forme de la composition s'effectue au moyen d'un instrument d'extrusion à une pression de filière de 150 bar et au-dessus.

2. Procédé selon la revendication 1, **caractérisé en ce que** la mise en forme de la composition s'effectue au moyen d'un instrument d'extrusion à angle, réduit en diamètre.

3. Utilisation de tuyaux médicaux fabriqués selon la revendication 1 ou 2, en particulier en tant que tuyaux transparents pour des systèmes conduisant du sang, pour des segments de pompe et pour le traitement sur des automates de confection.
